# EUROPÄISCHE PATENTSCHRIFT

(11) **EP 0 915 682 B1**
(45) Veröffentlichungstag und Bekanntmachung des Hinweises auf die Patenterteilung: **01.10.2003**
(21) Anmeldenummer: 97931780.7
(22) Anmeldetag: 04.07.1997
(51) Int. Cl.: A61B 18/12

(54) **GASUNTERSTÜTZTE, AXIAL VERSCHIEBBARE CHIRURGISCHE ELEKTRODE**
GAS-AIDED, AXIALLY DISPLACEABLE SURGICAL ELECTRODE
ELECTRODE CHIRURGICALE DEPLACABLE AXIALEMENT, ASSISTEE PAR UN GAZ

(30) Priorität: 04.07.1996 DE 19626976
(43) Veröffentlichungstag der Anmeldung: 19.05.1999
(73) Patentinhaber: Erbe Elektromedizin GmbH., D-72072 Tübingen (DE)
(72) Erfinder: FARIN, Günter, D-72070 Tübingen (DE); FISCHER, Klaus, D-72202 Nagold (DE); BARTEL, Volker, D-72810 Gomaringen (DE)
(74) Vertreter: Kruspig, Volkmar, Dipl.-Ing.
(86) Internationale Anmeldenummer: EP9703552
(87) Internationale Veröffentlichungsnummer: WO98001075

(56) Entgegenhaltungen:
- DE-A- 3 642 077
- DE-A- 4 222 769
- DE-A- 19 537 897
- US-A- 5 098 430
- US-A- 5 306 238

## Beschreibung

Die Erfindung betrifft ein chirurgisches Instrument vorzugsweise für die Hochfrequenzchirurgie gemäß Oberbegriff des Patentanspruches 1.

Chirurgische Instrumente für die Hochfrequenzchirurgie sind in verschiedenen Ausführungen seit längerer Zeit bekannt siehe zum Beispiel Dokument US-A-5 306 238. Die US-PS 5,007,008 beschreibt ein elektrisches chirurgisches Instrument, welches an seinem distalen Ende mit einer monopolaren oder bipolaren Koagulationselektrode ausgestattet ist, aus welcher bei Bedarf eine zum Schneiden geeignete Nadelelektrode herausgeschoben werden kann. Ein Nachteil dieses Instruments ist, daß der Operateur die Nadelelektrode manuell aus dem Instrument herausbewegen und gleichzeitig den Schneidmodus des verwendeten HF-Chirurgieinstruments aktivieren muß, so daß die Handhabung insgesamt erschwert ist.

Die DE-Patentanmeldung 195 37 897.0 sieht ein Multifunktionselement vor, bei dem unterschiedliche, einzeln ansteuer- und aktivierbare Applikatoren, Schneid- und Koagulationselektroden, eine Laser- sowie eine Ultraschallsonde und dgl. revolverartig in dem Gerät angeordnet sind. Die Bewegung des einzelnen Applikators zum distalen Ende des Instruments in Arbeitsstellung erfolgt über einen piezoelektrischen, elektromagnetischen und/oder hydraulischen Aktor. Auch kann der Aktor über ein Spracherkennungsmodul direkt angesprochen werden. Ein solches multivalentes Gerät ist jedoch kostenintensiv.

Der Erfindung liegt daher die Aufgabe zugrunde, ein einfaches chirurgisches Instrument vorzugsweise für die HF-Chirurgie vorzuschlagen, das kostengünstig herzustellen und einfach handhabbar ist.

Der Grundgedanke der Erfindung besteht darin, ein chirurgisches Instrument vorzugsweise für die HF-Chirurgie anzugeben, dessen Funktionen mit einer Hand gesteuert werden können.

Hierfür weist das Instrument eine Handhabungseinrichtung, ein Hüllrohr, Anschlüsse für HF-Strom und Gas und eine auswechselbare Elektrode an seinem distalen Ende auf.

Die Steuerung des Instruments, d.h. das Heraus- und Hereinfahren der Elektrode aus dem Hüllrohr bzw. das Zurückziehen und Vorfahren des Hüllrohres und der Wechsel von einem Schneid- zum APC (engl.: Argon-Plasma-Coagulation)-Modus wird durch die axial angeordnete Handhabungsvorrichtung, die im wesentlichen aus einem Haltegriff und einem bewegbaren Griff besteht, erreicht.

Dabei liegt der Haltegriff sicher in der Handfläche des Operateurs und der Daumen am bewegbaren Griff. Durch Bewegung des Daumens wird der Griff derart bewegt, daß die Elektrode axial in Arbeitsstellung oder ins Hüllrohr verbracht werden kann. Durch Federkraftverspannung kann ein automatisches Zurückbewegen der Elektrode in das Hüllrohr erfolgen, so daß Verletzungen ausgeschlossen oder Beschädigungen der Elektrode verhindert werden. Alternativ oder in Kombination kann mittels axialer Verschiebung des Hüllrohres, das mit dem Griff verbunden ist, die Elektrode freigelegt oder abgedeckt werden.

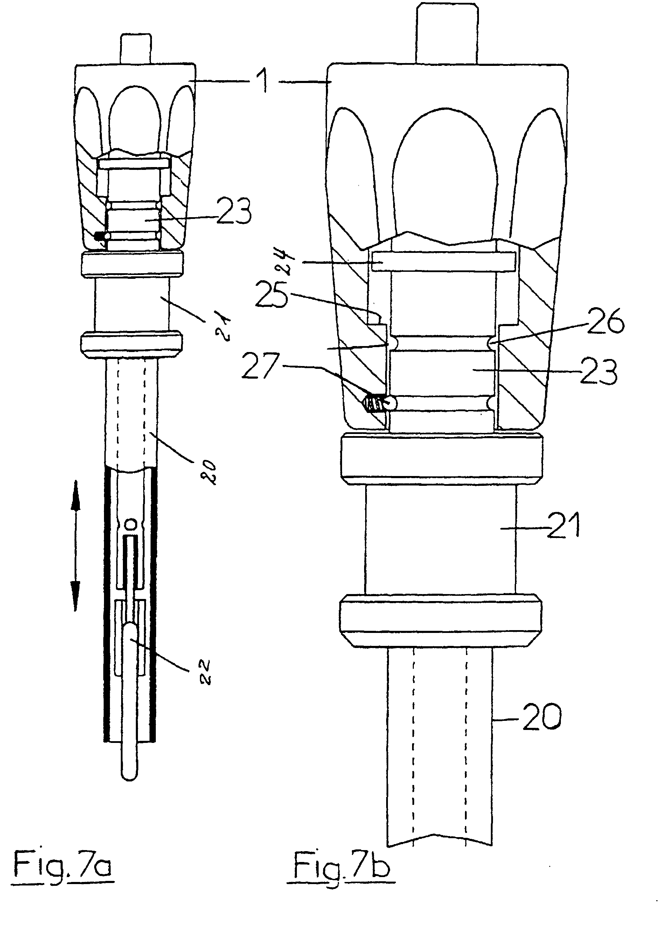

Die Erfindung soll anhand von Zeichnungen und Ausführungsbeispielen näher erläutert werden. Nur die Ausführungsform gemäß Fig. 7a and 7b zeigt die beanspruchte Erfindung.

Hierbei zeigen:
- Fig. 1: ein erstes Ausführungsbeispiel des chirurgischen Instruments,
- Fig. 2a und 2b: ein weiteres Ausführungsbeispiel des chirurgischen Instruments mit auswechselbarer Elektrode,
- Fig. 3 und 4: weitere Ausführungsbeispiele des chirurgischen Instruments mit abgewinkeltem Hüllrohr,
- Fig. 5 und 6: zwei Ausführungsbeispiele mit bewegbarer Elektrodenhalterung und
- Fig. 7a und 7b: eine Ausführungsform mit verschiebbarem Hüllrohr bzw. Schutzhülse.

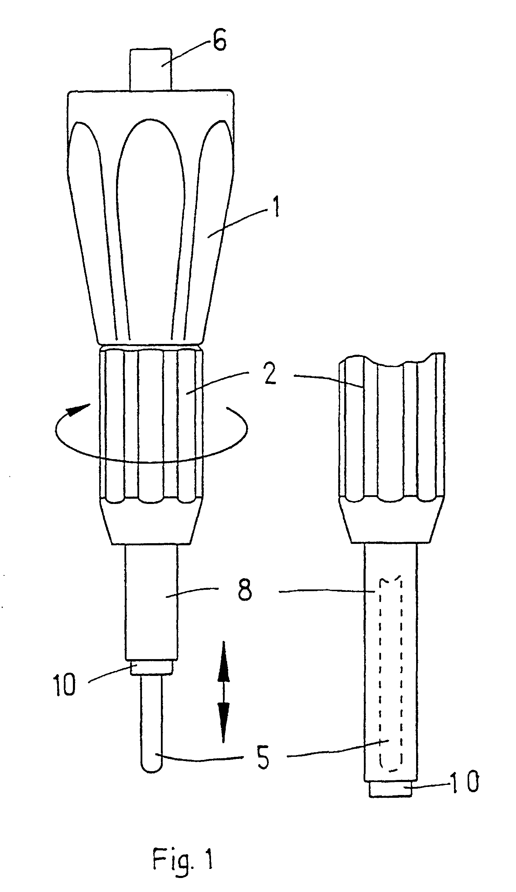

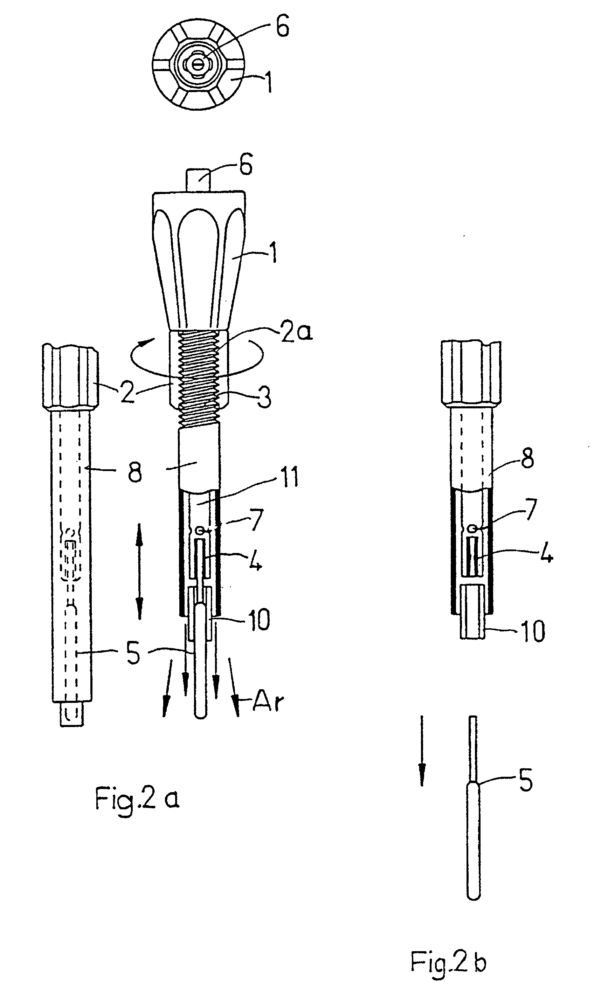

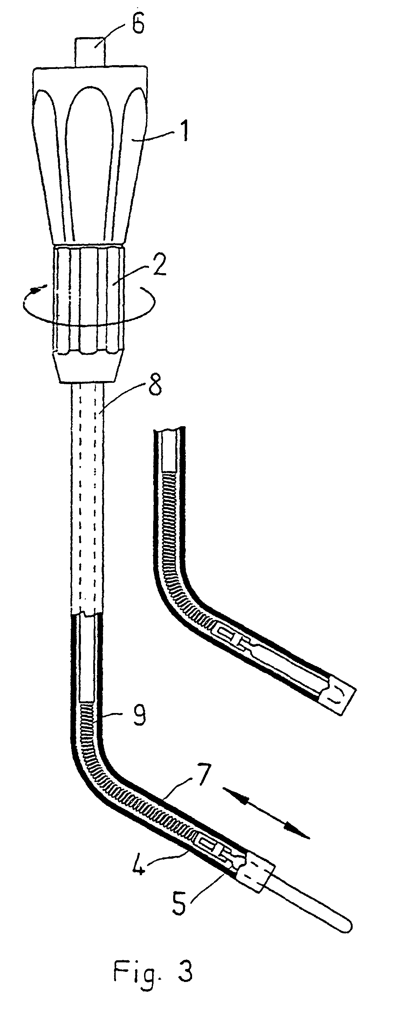

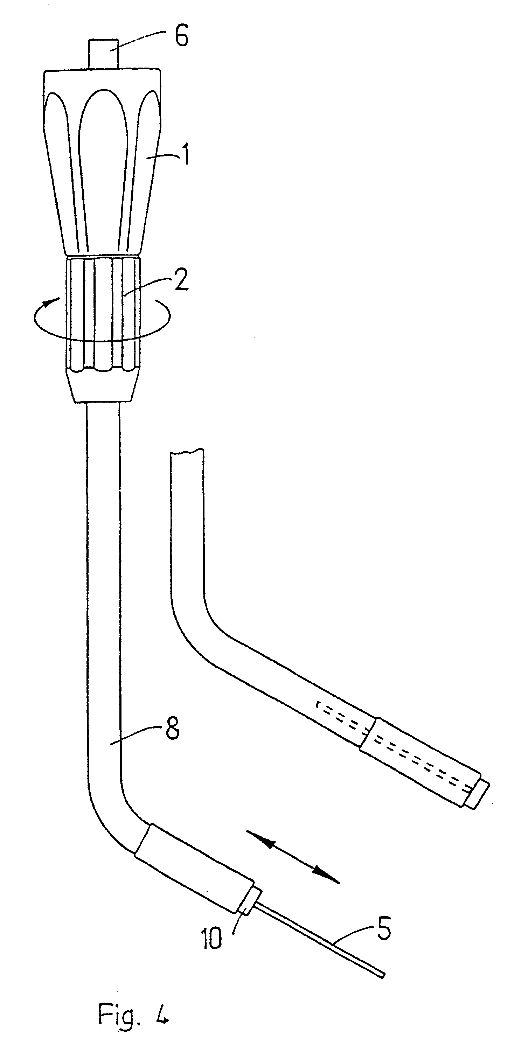

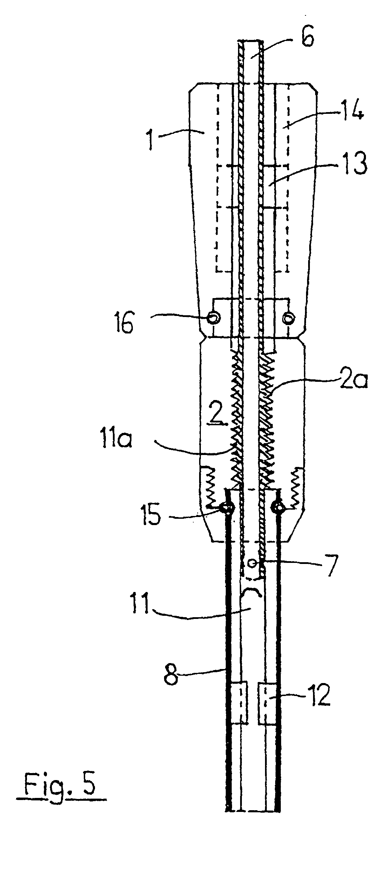

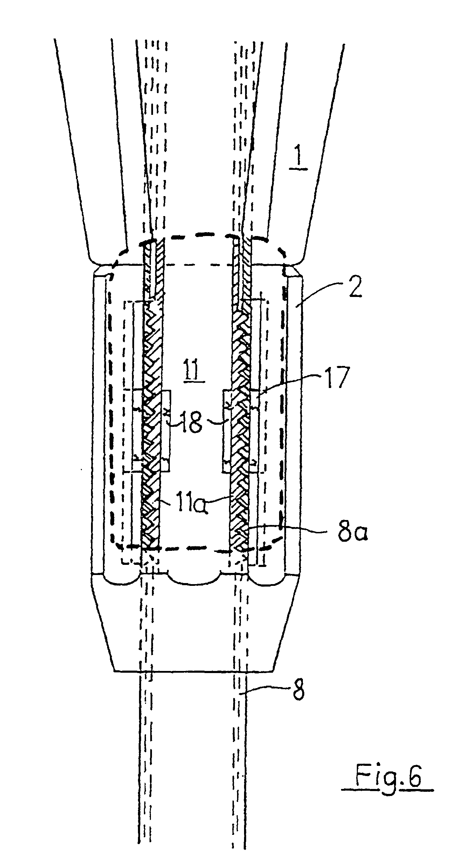

Das chirurgische Instrument gemäß Fig. 1, 2a und 2b umfaßt eine Elektrode 5 (in verschiedenen Ausführungsformen, z.B. als Nadel, Fig. 4, oder als Spatel, Fig. 2 und 3) befestigt mit einer Rast- oder Klemmvorrichtung 4 oder dgl. an einer Elektrodenhalterung 11, ein die Elektrode 5 und Halterung 11 umschließendes Hüllrohr 8 mit einem Außengewinde 3 am griffseitigen Ende sowie eine Handhabungsvorrichtung mit Gas- und HF-Strom-Anschluß, die mit Halterohr 8 und Elektrodehalterung 11 verbunden ist.

Die Handhabungsvorrichtung besteht aus einer axialen Anordnung eines Haltegriffs 1 mit dem Gas- und HF-Strom-Anschluß 6 und kann in ein nicht gezeigtes Griffstück eingesetzt werden. An dem Haltegriff 1 schließt sich distal ein Drehgriff 2 mit einem Innengewinde 2a an, das dem Außengewinde 3 des Hüllrohrs 8 entspricht und mit diesem in Eingriff steht.

Die Elektrodenhalterung 11 ist im Haltegriff 1 befestigt.

Bei der Anwendung des chirurgischen Instruments liegt dieses mit dem Haltegriff 1 und Griffstück in der Handfläche des Operateurs, so daß dieser mit dem Daumen den Drehgriff 2 bewegen kann. Das Gewinde 2a, 3 ist so ausgelegt, daß etwa mit einer Vierteldrehung des Drehgriffs 2 die Elektrode 5 zum Schneiden freigegeben wird, indem das Hüllrohr 8 von der Elektrode zurückbewegt wird. Mit einer entgegengesetzten Daumenbewegung wird das Hüllrohr 8 wieder über die Elektrode 5 bewegt, diese ist dann geschützt. Gleichzeitig mit der Elektrodenbewegung ist ein Ein- oder Umschalten des Betriebsmodus des Instruments realisierbar.

In einer weiterbildenden Ausführungsform des Instruments ist der Drehgriff 2 mit einer Federkraft beaufschlagt, derart, daß bei Freigabe der Elektrode die Feder gespannt wird. Wird ein Rastknopf oder der Drehgriff wieder freigegeben, so bewegt sich das Hüllrohr selbsttätig wieder über die Elektrode.

Ein drittes Ausführungsbeispiel sieht demnach am Drehgriff 2 ein Feststellelement in Form des Rastknopfes oder dergleichen (nicht gezeigt) vor, mittels dessen durch Daumendruck die gespannte Feder arretiert und wieder gelöst werden kann.

Ein viertes Ausführungsbeispiel betrifft die Ausbildung des griffseitigen Endes der Elektrodenhalterung 11 als Spindel oder dgl., in deren Gewinde 11a das Innengewinde 2a des Drehgriffs 2 eingreift, so daß die Elektrode 5 in das Hüllrohr 8 hinein- oder aus diesem herausbewegt werden kann.

Ein fünftes Ausführungsbeispiel geht von einem am distalen Ende abwinkelbaren Hüllrohr 8 (Fig. 3 und 4) aus, so daß schwer zugängliche Operationsfelder leichter erreicht werden können. Hierfür ist die Elektrodenhalterung 11 flexibel, vorzugsweise als biegsame Wendel 9 ausgebildet.

Bei einem sechsten Ausführungsbeispiel gemäß Fig. 5 ist die Elektrodenhalterung 11 griffseitig mit einem Außengewinde 11a versehen, das mit dem Innengewinde 2a des Drehgriffs 2 in Eingriff steht. Zur Führung der Elektrodenhalterung 11 ist diese griffseitig mit mindestens zwei radial abstehenden Flügeln 13 versehen, die sich in einer entsprechenden Führungsnut 14 im Haltegriff (1) axial bewegen.

Distal ist die sonst rohrförmige Elektrodenhalterung 11 als Flachstück ausgebildet, das sich in zwei Paaren von Führungsschienen 12 axial bewegt, welche im Hüllrohr 8 angeordnet sind. Um die Drehbarkeit des Drehgriffs 2 gegenüber dem Hüllrohr 8 einerseits und dem Haltegriff 1 andererseits zu gewährleisten, sind an den betreffenden Stellen Lager, vorzugsweise Kugellager oder Gleitlager 15, 16 angeordnet. Ein Edelgas, z.B. Argon wird am griffseitigen Ende der Elektrodenhalterung 11, welches aus dem Haltegriff 1 als Stutzen 6 herausragt, eingeleitet und strömt durch die Bohrungen 7 in das Hüllrohr und umströmt von dort aus die Elektrode 5 und das entsprechende Operationsfeld.

Ein siebentes Ausführungsbeispiel gemäß Figur 6 umfaßt die Bewegung der Elektrode mittels einer im Drehgriff 2 angeordneten Magnetomechanik. Dazu ist im Drehgriff 2 eine zylindrische Hinterschneidung axial ausgebildet, in der axial beweglich mindestens zwei Permanentmagnetstreifen 17 angeordnet sind. Diese Magnetstreifen werden in radialen Führungsnuten geführt.

Im Hüllrohr 8, welches im Haltegriff 1 befestigt ist, ist im Bereich des Drehgriffs 2 ein Innengewinde 8a ausgebildet, das mit einem Außengewinde 11a auf der rohrförmigen Elektrodenhalterung 11 in Eingriff steht.

In der Elektrodenhalterung 11 sind gegenüber den beweglichen Magneten 17 weitere Magnete 18 befestigt. Wird der Drehgriff 2 mit den Magneten 17 gedreht, werden durch die Wirkung der Magnetfelder die Magneten 18 in der Elektrodenhalterung 11 mitgezogen. Die Gewinde 8a, 11a bewirken, daß durch die Rotationsbewegung die Elektrodenhalterung 11 sich in axialer Richtung bewegt. An Stelle der Magnetstreifen können auch kreisringförmige oder kreissegmentförmige Magneten verwendet werden.

Beim siebten Ausführungsbeispiel gemäß den Figuren 7a und 7b wird von einem axial verschieblichen Hüllrohr in Form einer Schutzhülse 20 ausgegangen. Die Schutzhülse weist eine Griffmulde oder einen Griffring 21 auf. Über einen Fortsatz 23 ist der Griffring 21 und damit die Schutzhülse 20 in einer Führungsbohrung des Haltegriffes 1 beweglich gelagert. Ein umlaufender Bund 24 sorgt in Verbindung mit einem Anschlag 25 innerhalb des Haltegriffes 1 für eine Begrenzung des axialen Verschiebeweges der Schutzhülse 20, so daß die Elektrode 22 abgedeckt und freigelegt werden kann, ohne daß die Gefahr des unerwünschten vollständigen Abziehens der Schutzhülse 20 besteht. Dies erfolgt durch Verschiebung der Schutzhülse 20 mittels Krafteinwirkung im Bereich des Griffringes 21. Zum Erhalt vorgebbarer Rastpostionen der Schutzhülse 20 besitzt der Fortsatz 23 Rastausnehmungen 26, beispielsweise in Form einer umlaufenden Nut. In diese Nut greifen dann federbelastete Raststifte oder Rastkugeln 27 ein. Selbstverständlich ist auch eine kinematische Umkehr der Anordnung von Rastausnehmungen und Raststiften oder Rastkugeln denkbar.

Das vorgeschlagene chirurgische Instrument ist leicht herstellbar, einfach und sicher in der Handhabung. Die Sicherung der Schneid- oder Koagulationselektrode durch ein mögliches Zurückbewegen ins Hüllrohr schützt zum einen die Elektrode, und zum anderen wird einer möglichen Verletzungsgefahr vorgebeugt.

### Bezugszeichenliste

- 1: Haltegriff
- 2: Drehgriff
- 2a: Innengewinde
- 3: Außengewinde
- 4: Klemmvorrichtung
- 5, 22: Elektrode
- 6: Gasanschluß
- 7: Gasaustrittsbohrungen
- 8: Hüllrohr
- 8a: Innengewinde des Hüllrohrs
- 9: Federwendel
- 10: Isolator
- 11: Elektrodenhalterung
- 11a: Außengewinde der Elektrodenhalterung
- 12: Führungsschienen
- 13: Flügel
- 14: Führungsnut
- 15: Kugellager
- 16: Kugellager
- 17: Magnete
- 18: Magnete
- 20: Schutzhülse
- 21: Griffring
- 22: Fortsatz
- 24: Bund
- 25: Anschlag
- 26: Rastausnehmungen
- 27: Rastkugeln

## Patentansprüche

1. Chirurgisches Instrument, vorzugsweise für die Hochfrequenzchirurgie, umfassend
- eine Handhabungsvorrichtung;
- einen Gas- und einen Hochfrequenzstromanschluss;
- ein Hüllrohr (20);
- eine Elektrode (22), die an einer Elektrodenhalterung im Hüllrohr angeordnet ist,
- wobei die Handhabungsvorrichtung aus einer axialen Anordnung eines Haltegriffs (1) und eines sich distal anschließenden, relativ gegen den Haltegriff (1) bewegbaren Griff mit einem Griffring (21) besteht;
- das Hüllrohr (20) mit dem Griffring (21) oder Griff verbunden ist, so dass durch eine axiale Verschiebung des Griffs das Hüllrohr (20) axial nach vorn oder nach hinten zum Freilegen oder Abdecken der Elektrode (22) bewegt werden kann, und
- im Hüllrohr (20) die Elektrode (22) an einem Halteelement befestigt ist, das mit dem Haltegriff (1) verbunden ist,
**dadurch gekennzeichnet, dass**
der Haltegriff (1) eine Führungsbohrung zur Aufnahme eines Fortsatzes (23) aufweist, welcher mit dem Griffring (21) und/oder dem eine Schutzhülse bildenden Hüllrohr (20) verbunden ist, innerhalb der Führungsbohrung ein Anschlag (25) ausgebildet ist, welcher mit einem umlaufenden Bund (24) des Fortsatzes (23) eine Verschiebesicherung oder Verschiebebegrenzung bildet und wobei zum Erhalt vorgebbarer Rastpositionen der Schutzhülse der Fortsatz (23) Rastausnehmungen (26) aufweist, die mit Raststiften oder Rastkugeln (27) des Haltegriffs (1) wechselwirken.

## Claims

1. A surgical instrument, preferably for use in the high frequency surgery, comprising
- a manipulating apparatus;
- a gas and a high frequency current connection;
- an enveloping tube (20);
- an electrode (22) which is arranged at an electrode mounting in the enveloping tube;
- wherein the manipulating apparatus consists of an axial arrangement of a holding grip (1) and a distally connected grip which is movable relative to the holding grip (1) with a gripping ring (21);
- the enveloping tube (20) is connected with the gripping ring (21) or the grip so that the enveloping tube (20) can be axially advanced or retracted for exposing or covering the electrode (22) by an axial displacement of the grip, and
- the electrode (22) in the enveloping sleeve (20) is attached at a mounting element which is connected with the holding grip (1),
**characterised in that** the holding grip (1) comprises a guide hole for accommodating an extension (23) which is connected with the gripping ring (21) and/or the enveloping tube (20) forming a protecting sleeve, a stop (25) is formed within the guide hole, which together with a circumferential shoulder (24) of the extension (23) forms an anti-displacement lock or a displacement barrier, and wherein the extension (23) comprises locking recesses (26) which interact with locking pins or locking balls (27) of the holding grip (1) for obtaining predeterminable locking positions of the protective sleeve.

## Revendications

1. Instrument chirurgical, de préférence pour la chirurgie à hautes fréquences, comportant :
- un dispositif de manipulation,
- un raccordement de gaz et un raccordement électrique à haute fréquence,
- un tube enveloppe (20),
- une électrode (22) qui est agencée sur un porte-électrode dans le tube enveloppe,
- le dispositif de manipulation étant constitué par un agencement axial d'une poignée de retenue (1) et d'une poignée qui se raccorde du côté distal, qui est mobile par rapport à la poignée de retenue et qui comprend un anneau de poignée (21),
- le tube enveloppe (20) étant relié à l'anneau de poignée (21) ou à la poignée, de sorte que suite à une translation axiale de la poignée le tube enveloppe (20) peut être déplacé axialement vers l'avant ou vers l'arrière pour dégager ou recouvrir l'électrode (22), et
- dans le tube enveloppe (20), l'électrode (22) est fixée sur un élément de retenue qui est relié à la poignée de retenue (1),
**caractérisé en ce que**
la poignée de retenue (1) comprend un perçage de guidage pour recevoir un prolongement (23) qui est relié à l'anneau de poignée (21) et/ou au tube enveloppe (20) formant une gaine de protection, **en ce qu'**une butée (25) est réalisée à l'intérieur du perçage de guidage, qui forme conjointement avec un collier périphérique (24) du prolongement (23) un blocage anti-translation ou une limitation de translation, et **en ce que** pour obtenir des positions d'enclenchement prédéterminées de la gaine de protection, le prolongement (23) présente des évidements d'enclenchement (26) qui coopèrent avec des tiges d'enclenchement ou des billes d'enclenchement (27) de la poignée de retenue (1).
